# EUROPÄISCHE PATENTSCHRIFT

(11) **EP 1 800 118 B1**
(45) Veröffentlichungstag und Bekanntmachung des Hinweises auf die Patenterteilung: **23.10.2013**
(21) Anmeldenummer: 05787316.8
(22) Anmeldetag: 16.09.2005
(51) Int. Cl.: G01N 31/22, C07D 231/46, C12Q 1/28

(54) **MITTEL UND VERFAHREN ZUM NACHWEIS VON FURFURALEN**
AGENT AND METHOD FOR IDENTIFYING FURFURALS
MOYEN ET PROCEDE D'IDENTIFICATION DE FURFURALS

(30) Priorität: 15.10.2004 DE 102004050209
(43) Veröffentlichungstag der Anmeldung: 27.06.2007
(73) Patentinhaber: Merck Patent GmbH, 64293 Darmstadt (DE)
(72) Erfinder: WAGNER, Berthold, 60489 Frankfurt (DE); BEIL-SEIDLER, Stefanie, 64546 Moerfelden-Walldorf (DE)
(86) Internationale Anmeldenummer: PCT/EP2005/009984
(87) Internationale Veröffentlichungsnummer: WO 2006/042600

(56) Entgegenhaltungen:
- EP-A- 0 469 445
- EP-A- 0 526 150
- DD-A7- 270 847
- GB-A- 1 593 735

## Beschreibung

Die Erfindung betrifft ein Mittel und ein Verfahren zum Nachweis von Furfuralen, insbesondere der chemischen Signalstoffe 5-(Hydroxymethyl)-furan-2-carbaldehyd (Hydroxymethylfurfural, HMF) und Furan-2-carbaldehyd (Furfural).

Eine Hitzebehandlung ist der gebräuchlichste Weg Lebensmittel zu konservieren und genießbar zu machen. Unter genau kontrollierten Bedingungen behalten die Lebensmittel ihre ureigensten, organoleptischen und ernährungsphysiologischen Eigenschaften unter der Hitze/Wärmebehandlung. Jede über das nötige Maß hinausgehende Hitzebehandlung kann Lebensmittelinhaltsstoffe verändern und damit einhergehend den Geschmack und Nährwert negativ zu beeinflussen. Daher werden häufig chemische Signalstoffe zur Qualitätsbeurteilung von Lebensmitteln und zur Steuerung und Optimierung von Prozessen in der Lebensmittelverarbeitung eingesetzt.

Hydroxymethylfurfural (HMF) ist ein bekannter Indikator für den Qualitätsverlust durch exzessive Hitzebehandlung oder Lagerung von zuckerhaltigen Lebensmitteln.

Der HMF Gehalt von frischen, unbehandelten Fruchtsäften ist praktisch null und die Bestimmung des HMF Gehaltes ist eine gängige Methode, um die Effektivität einer Hitzebehandlung zur Inaktivierung unerwünschter Mikroorganismen in Konfitüren und Fruchtzubereitungen zu beurteilen.

HMF ist ein Hauptprodukt der Maillard Reaktion und wird bei der Reaktion von reduzierenden Zuckern mit Aminosäuren in Abhängigkeit von Temperatur und pH-Wert gebildet. In Untersuchungen von unterschiedlichen Lebensmitteln wurden Konzentrationen von mehr als 1g /kg HMF in getrockneten Früchten, Karamell-Produkten und Säften, die aus Trockenpflaumen hergestellt wurden, gefunden.

Die International Federation of Fruit Juice Processors (IFFJP) empfiehlt eine Höchstkonzentration von 5-10 mg/l HMF in Fruchtsäften und 25 mg/kg in Fruchtsaftkonzentraten .

In Tomatensäften und Tomatenmark wurden in Abhängigkeit vom Zucker- und Feststoffgehalt (Brix-Wert) 1,3 bis 186 mg/ kg HMF gefunden.

In frischen Honigen ist HMF mit Gehalten von unter 1 mg/kg nachweisbar und Honig ist ab einem Gehalt von 15 mg/kg nur noch als Industriehonig verwertbar.

Auch in der Milchverarbeitung, insbesondere der Ultrahocherhitzung, hat der HMF Gehalt einige Bedeutung. Allerdings müssen hierbei die schwankenden HMF-Gehalte in der Rohmilch beachtet werden.

Der HMF Gehalt hat neben seiner Bedeutung als Erhitzungsparameter in Lebensmittel einen nicht zu vernachlässigenden, direkten Einfluss auf den Geruch und den Geschmack der Lebensmittel.

Die Bestimmung des HMF Gehaltes von Lebensmitteln und Rohstoffen der Lebensmittelverarbeitung hat Eingang in die Routineanalytik der Qualitätskontroillaboratorien gehalten. Es finden verschiedene Methoden Anwendung, die alle einer Probenvorbereitung, wie Destillation oder Extraktion, bedürfen. Dazu gehören die Gaschromatographie, HPLC oder spektrophotometrische Nachweise mit der Methode nach Winkler (Winkler, 1955, Z. Lebensm.-Untersuch.-Forsch., 102,161), bzw. gemäß der A.O.A.C. Methode.

Alle diese Methoden bedürfen eines hohen apparativen Aufwandes, wie auch geschulten Personals zur sicheren Durchführung.

Im übrigen sind diese Methoden nicht geeignet zur schnellen Vor-Ort Analytik, die es gewährleisten würde, die Verarbeitungsprozesse zeitnah zur Analytik zu steuern.

Die am häufigsten angewandte Methode zur HMF Bestimmung nach Winkler (empfohlen in Richtlinien der E.U. und O.I.V.) arbeitet mit giftigem p-Toluidin (CAS-Nr. 106-49-0), welches neben der Gesundheitsgefährdung des unerfahrenen Anwenders und der Entsorgungsproblematik der anfallenden Reststoffe, auch eine Anwendung in räumlicher Nähe zur Lebensmittelverarbeitung ausschließt. Zudem birgt die Methode nach Winkler das Problem, dass die entstehende Färbung nur kurze Zeit stabil ist und so die Auswertung erschwert.

Dem Ersatz der HMF-Bestimmungsmethode nach Winkler und der Bereitstellung eines anwenderfreundlichen und kostengünstigen Schnelltests zur HMF Analytik, insbesondere in Lebensmitteln, kommt eine hohe Bedeutung zu.

Der Erfindung lag daher die Aufgabe zugrunde, ein Mittel zum Nachweis von HMF zur Verfügung zu stellen, das ohne großen apparativen Aufwand direkt vor Ort angewendet werden kann.

Es wurde gefunden, dass eine Reagenzkombination aus einem 4-Aminophenazon-Derivat, insbesondere 4-Aminophenazon (4-Aminoantipyrin, CAS-Nr. 83-07-8) und einem Barbitursäure-Derivat, insbesondere Barbitursäure (CAS-Nr. 67-52-7) oder Thiobarbitursäure (CAS-Nr. 504-17-6) im Sauren in Anwesenheit von HMF eine rot-violette Färbung ergibt. Diese Färbung kann visuell oder photometrisch ausgewertet werden. Insbesondere kann die Reagenzkombination auch in Form eines Teststreifens vorliegen, so dass die Durchführung des HMF-Nachweises besonders einfach möglich ist.

Weiterhin wurde gefunden, dass mit derselben Reagenzkombination auch der Nachweis von anderen Furfuralen, wie z.B. an der 5-Position substituierten Furfural-Derivaten und Furfural selbst möglich ist. Zudem kann eine Unterscheidung zwischen HMF und Furfural getroffen werden, da bei Einsatz von 4-Amino-Benzoesäure in Anwesenheit von Furfural eine blaue Färbung auftritt, während HMF dagegen keine Färbung hervorruft.

Gegenstand der vorliegenden Erfindung ist daher ein Mittel zum Nachweis wie in Anspruch 1 definiert.

In einer anderen bevorzugten Ausführungsform enthält das Mittel 4-Aminophenazon als 4-Amino-phenazon-Derivat.

In einer weiteren bevorzugten Ausführungsform enthält das Mittel Barbitursäure und/oder Thiobarbitursäure als Barbitursäure-Derivat.

In einer weiteren bevorzugten Ausführungsform enthält das Mittel Citronensäure.

In einer besonders bevorzugten Ausführungsform besteht das Mittel aus einem Teststreifen, der mit einer sauren Lösung getränkt wurde, die zumindest 0,1 bis 20 Gew.% 4-Aminophenazon und 0,05 bis 5 Gew.% Barbitursäure und/oder Thiobarbitursäure enthält.

In einer anderen Ausführungsform besteht das Mittel aus einem Teststreifen, der zusätzlich eine Zone aufweist, die mit einer sauren Lösung getränkt wurde, die zumindest 4-Aminobenzoesäure und ein Barbitursäure-Derivat enthält.

Gegenstand der vorliegenden Erfindung ist auch ein Verfahren zum Nachweis von Furfuralen, gekennzeichnet durch folgende Verfahrensschritte
a) Bereitstellen einer wässrigen Probenlösung
b) Inkontaktbringen des erfindungsgemäßen Mittels, wie in Anspruch 1 definiert, mit der Probenlösung aus Schritt a)
c) Visuelle und/oder photometrische Auswertung der entstehenden Färbung.

In einer weiteren bevorzugten Ausführungsform erfolgt die Auswertung in Schritt c) reflektometrisch.

Gegenstand der vorliegenden Erfindung ist auch die Verwendung des erfindungsgemäßen Mittels zum Nachweis von 5-(Hydroxymethyl)-furan-2-carbaldehyd in Lebensmitteln.

Gegenstand der vorliegenden Erfindung ist auch ein Kit zum Nachweis von Furfuralen zumindest enthaltend ein erfindungsgemäßes Mittel nach Anspruch 1.

In einer Ausführungsform enthält der Kit zusätzlich einen Teststreifen, der mit einer sauren Lösung getränkt wurde, die zumindest 4-Aminobenzoesäure und ein Barbitursäure-Derivat enthält.

Kern der vorliegenden Erfindung ist, dass eine Kombination aus einem Barbitursäure-Derivat und einem 4-Amino-phenazon-Derivat in saurer Lösung in Anwesenheit von Furfuralen, insbesondere 5-(Hydroxymethyl)-furan-2-carbaldehyd und/oder Furan-2-carbaldehyd, eine intensive, im allgemeinen rot bis violett-blaue Färbung ergeben. In Anwesenheit von 5-(Hydroxymethyl)-furan-2-carbaldehyd entsteht eine eher rot-violette Färbung während in Anwesenheit von Furan-2-carbaldehyd eine eher blauviolette Färbung entsteht. Je nach Art der eingesetzten Barbitursäure-Derivate und 4-Amino-phenazon-Derivate kann die Farbe variieren.

Eine Probenlösung ist jede Art von wässriger oder zumindest vorwiegend wässriger Lösung. Dabei kann es sich um eine unverdünnte Probe handeln wie z.B. im Fall von Säften oder anderen Getränken wie Bier oder Wein. Genauso kann die Probe jedoch zuerst mit Wasser oder einer Pufferlösung verdünnt bzw. gelöst werden. Dies ist beispielsweise bei hochkonzentrierten oder zäh- bzw. nicht-flüssigen Proben, wie z.B. Konfitüren oder Honig notwendig.

Im Fall von Proben, die nicht direkt mit Wasser mischbar sind, kann zunächst eine Extraktion vorgenommen werden. Dies ist z.B. zum Nachweis von 5-(Hydroxymethyl)-furan-2-carbaldehyd und/oder Furan-2-carbaldehyd in Ölen notwendig. Der Fachmann ist in der Lage, einen oder mehrere Extraktionsschritte mit Wasser und/oder mit Wasser mischbaren Lösungsmitteln und/oder wässrigen Pufferlösungen durchzuführen.

In jedem Fall ist es vorteilhaft, wenn die schlussendlich für den Nachweis eingesetzte Probenlösung eine wässrige, klare und nicht zu stark gefärbte Lösung ist. Es wurde weiterhin gefunden, dass die Gegenwart von Sulfit den erfindungsgemäßen Nachweis stört.

Bei sehr stark basischen oder stark, insbesondere basisch, gepufferten Probelösungen kann vor der Durchführung des erfindungsgemäßen Nachweises ein Ansäuern notwendig sein, da die erfindungsgemäße Farbreaktion nur im Sauren auftritt. Im allgemeinen reicht es jedoch aus, die Proben ohne vorheriges Ansäuern mit dem erfindungsgemäßen Mittel in Kontakt zu bringen.

4-Amino-phenazon-Derivate im Sinne der vorliegenden Erfindung sind 4-Amino-phenazon sowie Derivate dieser Verbindung, die an der 1- und/oder 5-Position statt der Methylgruppe eine andere Substitution, vorzugsweise einen C₂ bis C₅-Alkylrest, tragen. Genauso kann der Phenylring ein- oder mehrfach z.B. mit C₁ bis C₅-Alkylresten substituiert sein. In einer bevorzugten Ausführungsform wird als 4-Amino-phenazon-Derivat 4-Amino-phenazon selbst eingesetzt.

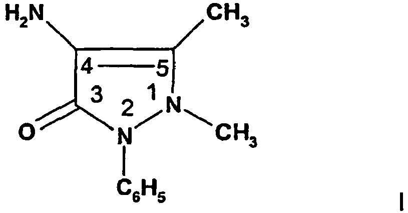

Formel I zeigt beispielhaft das erfindungsgemäß bevorzugte 4-Aminophenazon.

Barbitursäure-Derivate sind Barbitursäure und Thiobarbitursäure selbst, sowie Derivate, die an einem oder beiden Ring-Stickstoffatomen unabhängig voneinander C₁ bis C₆-Alkyl und/oder C₆ bis C₁₈-Aryl-Reste tragen, wie z.B. Methyl, Ethyl, Isopropyl, Cyclohexyl oder Phenyl. Ein Beispiel für ein derartiges Barbitursäurederivat ist 1,3-Dimethylbarbitursäure.

In einer bevorzugten Ausführungsform werden erfindungsgemäß Barbitursäure und/oder Thiobarbitursäure verwendet.

Erfindungsgemäß bedeuten die Begriffe "4-Amino-phenazon-Derivat" und "Barbitursäure-Derivat", dass jeweils einzelne Verbindungen oder eine Mischung derartiger Verbindungen zum Einsatz kommen.

Das erfindungsgemäße Mittel liegt in Form eines Teststreifens vor.

Eine Reagenzienlösung enthält typischerweise zumindest 0,1 bis 20 % (Gew.%), vorzugsweise 3 bis 8%, insbesondere ca. 5% des 4-Amino-phenazon-Derivats und 0,05 bis 5 % (Gew.%), vorzugsweise ca. 0,2%, des Barbitursäure-Derivats in einem sauren wässrigen Lösemittel. Das wässrige Lösemittel kann Wasser oder ein wässriges Puffersystem sein, das bis zu 75% (Vol.%) eines mit Wasser mischbaren Lösungsmittels wie z.B. Ethanol enthalten kann. In einer bevorzugten Ausführungsform besteht das Lösemittel aus gleichen Volumenteilen von Ethanol und einer sauren wässrigen Lösung. In einer besonders bevorzugten Ausführungsform liegt der pH-Wert des Lösemittels zwischen 2 und 5, besonders bevorzugt bei ca. 3,5. Zur Einstellung dieses pH-Wertes sind anorganische oder organischen Säuren und Basen geeignet. Beispielsweise kann eine wässrige Lösung aus 50g/l Citronensäure in Wasser hergestellt werden, deren pH-Wert mit Natronlauge auf pH 3,5 eingestellt wird.

Das erfindungsgemäße Mittel liegt in Form eines Teststreifens vor. Die Analyse mit festen, saugfähigen Trägern, den sogenannten Teststreifen, hat in den letzten Jahren zunehmend an Bedeutung gewonnen. Zu den wesentlichen Vorteilen dieser trockenchemischen Verfahren gehören insbesondere die einfache Handhabung sowie die aufgrund der geringen Reagenzmengen unproblematische Entsorgung. Alle oder der größte Teil der für die Nachweisreaktion notwendigen Reagenzien (farbgebendes Reagenz, Puffersystem, ggf. auch Stabilisatoren und Lösungsvermittler) sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge des zu bestimmenden Analyten und kann visuell, d.h. halbquantitativ durch Vergleich mit einer Farbkarte, bzw. quantitativ z.B. mit einfachen Reflektometern ausgewertet werden.

Als saugfähige Träger können alle Materialien verwendet werden, die üblicherweise für solche Tests im Gebrauch sind. Am weitesten verbreitet ist die Verwendung von Filterpapier, jedoch können auch andere saugfähige Cellulose-, Glasfaser- oder Kunststoffprodukte eingesetzt werden. Die Reagenzien können auch in transparente Trägerschichten eingebettet sein, deren filmbildenden Komponenten in Wasser quellbar sind. Die filmbildenden Komponenten können natürlichen Ursprungs, wie z.B.: Gelatine, Agarose, Alginat, oder synthetischen Ursprungs, wie z.B. Cellulosesester, Polyvinylacetat, Polyethylenimin, Polyvinylalkohol, sein. Die saugfähigen Träger werden in bekannter Weise mit Tränklösungen imprägniert, die die zur Bestimmung notwendigen Reagenzien enthalten. Die getränkten und getrockneten Träger, vorzugsweise Papiere, können entweder als solche in handliche Streifen geschnitten werden, oder sie können zu vorzugsweise quadratischen Zonen verarbeitet werden, die ihrerseits in bekannter Weise auf Kunststofffolien, Papier- oder Metallstreifen aufgeklebt bzw. auf- oder eingesiegelt werden können.

Die Tränklösung für das erfindungsgemäße Mittel in Form eines Teststreifens enthält typischerweise zumindest 0,1 bis 20 %, vorzugsweise 3% bis 8%, insbesondere ca. 5% eines 4-Amino-phenazon-Derivats und 0,05 bis 5 %, bevorzugt ca. 2% eines Barbitursäure-Derivats in einem sauren wässrigen Lösemittel. Das wässrige Lösemittel kann Wasser oder ein wässriges Puffersystem sein, das bis zu 75% (Vol.%) eines mit Wasser mischbaren Lösungsmittels wie z.B. Ethanol enthalten kann. In einer bevorzugten Ausführungsform besteht das Lösemittel aus gleichen Volumenteilen von Ethanol und einer sauren wässrigen Lösung. In einer besonders bevorzugten Ausführungsform liegt der pH-Wert des Lösemittels zwischen 2 und 5, besonders bevorzugt bei ca. 3,5. Zur Einstellung dieses pH-Wertes sind anorganische oder organischen Säuren und Basen geeignet. Beispielsweise kann eine wässrige Lösung aus 50g/l Citronensäure in Wasser hergestellt werden, deren pH-Wert mit Natronlauge auf pH 3,5 eingestellt wird.

In der bevorzugten Ausführungsform wird diese Tränklösung auf ein Folienband mit aufgesiegelter Filtrierpapierzone aufgetragen und im Warmluftstrom getrocknet.

Nach dem Aufschneiden in Teststreifen stehen diese für qualitative, wie auch quantitative Analysen zur Verfügung.

Gegenstand der vorliegenden Erfindung ist auch ein Verfahren zum Nachweis von Furfuralen, insbesondere 5-(Hydroxymethyl)-furan-2-carbaldehyd und/oder Furan-2-carbaldehyd, gekennzeichnet durch folgende Verfahrensschritte
a) Bereitstellen einer wässrigen Probenlösung

Dabei kann es notwendig sein, die zu analysierende Probe zunächst z.B. durch Verdünnen, Lösen oder Extraktion in eine geeignete Form zu überführen.
b) Inkontaktbringen des erfindungsgemäßen Mittels, wie in Anspruch 1 definiert, mit der Probenlösung aus Schritt a)

Das erfindungsgemäße Mittel wird mit der Probenlosung benetzt oder kurz in die Probenlösung getaucht. Hier kann, falls notwendig, die Probenlösung vorher entsprechend verdünnt oder aufkonzentriert werden. Typischerweise tritt innerhalb von 1 bis 10 Minuten in Abhängigkeit vom Analyt-Gehalt in der Probelösung eine Farbentwicklung auf, wobei die Farbentwicklung auf einem Teststreifen zumeist schon nach 1 bis 3 Minuten erfolgt.
c) Visuelle und/oder photometrische Auswertung der entstehenden Färbung.

Die Farbentwicklung kann durch Vergleich mit einer Farbkarte ausgewertet werden und/oder, bevorzugt, durch quantitative Analyse mit einem Reflektometer.

Das erfindungsgemäße Mittel und Verfahren eignet sich besonders gut für den Nachweis von HMF und/oder Furfural in Lebensmitteln. Die eingesetzten Reagenzien sind nicht giftig und die Teststreifen können auch von ungeschultem Personal direkt vor Ort eingesetzt werden. Für die quantitative Analyse sind außer einem kleinen Hand-Reflektometer keine weiteren Gerätschaften notwendig. Mit dem erfindungsgemäßen Mittel und Verfahren können typischerweise HMF-Konzentrationen zwischen 0,3 und 100 mg/l nachgewiesen werden.

Im Gegensatz zu der Methode nach Winkler, bei der die Farbentwicklung bereits nach wenigen Minuten verblasst, kann mit den erfindungsgemäßen Mitteln eine wesentlich bessere Farbstabilität erzielt werden. Die erfindungsgemäßen Reagenzlösungen zeigen zumeist eine Farbstabilität über mindestens 15 bis 30 Minuten. Der erfindungsgemäße Teststreifen ist besonders bevorzugt, da hier die Farbe typischerweise bereits nach 1 bis 3 Minuten entwickelt ist und danach für Stunden bis hin zu Tagen stabil bleibt. Bevorzugt wird das erfindungsgemäße Mittel daher als Teststreifen eingesetzt oder in Form eines Kits, der zumindest einen erfindungsgemäßen Teststreifen enthält. In einer bevorzugten Ausführungsform enthält der Kit zusätzlich eine Farbtafel sowie je nach Art der Proben Lösemittel zum Verdünnen oder Extrahieren der Proben.

Es wurde weiterhin gefunden, dass ein erfindungsgemäßes Mittel beim Ersatz des 4-Amino-phenazon-Derivats durch 4-Aminobenzoesäure in Anwesenheit von HMF keine Farbreaktion mehr zeigt, wohingegen in Anwesenheit von Furfural eine blaue Farbe auftritt. Auf diese Weise kann zwischen HMF und Furfural unterschieden werden, so dass sowohl HMF wie auch Furfural in einer Probe beide halbquantitativ und/oder quantitativ bestimmt werden können. Dazu wird die Probe mit einem erfindungsgemäßen Mittel analysiert und so der Gehalt an Furfuralen (typischerweise lediglich HMF und Furfural) bestimmt. Zusätzlich wird eine weitere Analyse mit einem Mittel durchgeführt, in dem das 4-Aminophenazon-Derivat durch 4-Aminobenzoesäure ersetzt ist. Dadurch kann der Gehalt an Furfural ermittelt werden. Aus der Differenz der beiden Ergebnisse ergibt sich dann der Gehalt an HMF.

Gegenstand der vorliegenden Erfindung ist daher auch ein Verfahren und Mittel zum parallelen Nachweis von HMF und Furfural in einer Probe.

Bei dem Mittel handelt es sich um einen Teststreifen, der in einer Zone eine Reagenzkombination aus zumindest einem 4-Amino-phenazon-Derivat und einem Barbitursäure-Derivat im Sauren enthält und in einer anderen Zone eine Reagenzkombination, die zumindest 4-Aminobenzoesäure und ein Barbitursäure-Derivat im Sauren enthält. Auf diese Weise kann der Nachweis parallel auf einem Teststreifen durchgeführt werden.

Genauso kann der parallele Nachweis von HMF und Furfural mithilfe eines Kits durchgeführt werden, der zumindest ein erfindungsgemäßes Mittel enthält, das zumindest ein 4-Amino-phenazon-Derivat und ein Barbitursäure-Derivat im Sauren enthält und ein Mittel, das zumindest 4-Aminobenzoesäure und ein Barbitursäure-Derivat im Sauren enthält. Dies können zwei Reagenzlösungen oder auch zwei Teststreifen sein.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die vollständige Offenbarung aller vor- und nachstehend aufgeführten Anmeldungen, Patente und Veröffentlichungen, insbesondere der korrespondierenden Anmeldung DE 102004050209.9, eingereicht am 15.10.2004, ist durch Bezugnahme in diese Anmeldung eingeführt.

### Beispiele

### 1. Erstellung einer Eichgeraden

### a) Herstellung der Reagenzienlösung

5% (Gew%) 4-Amino-phenazon und 0,2% (Gew%) Barbitursäure werden in einem Lösemittel gelöst. Das Lösemittel besteht aus gleichen Volumenteilen von Ethanol und einer Pufferlösung bestehend aus 50g/l . Citronensäure in Wasser; der pH-Wert des Lösemittels wird mit Natronlauge eingestellt auf 3,5. Wird die Reagenzienlösung mit einer HMF-Standardlösung in vollentsalztem Wasser im Volumenverhältnis 10/1 versetzt, so erbeben sich bei 20° C nach 15 min Reaktionszeit folgende Extinktionen (1 cm Schichtdicke, 550 nm):

**(Tabelle 1)**

| **HMF in mg/l** | **Extinktion** |
|---|---|
| 0 | 0,006 |
| 5 | 0,081 |
| 10 | 0,157 |
| 20 | 0,315 |
| 50 | 0,795 |
| 100 | 1,536 |

Für diese Werte generiert sich eine Gerade mit der Gleichung y= 0,0153x + 0,0077 und einem Korrelationskoefizienten von 0.9998.

Es wurden Realproben (beide mit HMF Standards kalibriert) vergleichend mit der Winkler-Methode und der erfindungsgemäßen Reagenzienlösung untersucht.

Dabei ergaben sich folgende Messwerte:

**(Tabelle 2, PIW bedeutet Blindwert)**

| | **Methode nach Winkler** | | | **Erfindungsgemäße Methode** | | |
|---|---|---|---|---|---|---|
| **Probe** | **Extinktion** | | **HMF** | **Extinktion** | | **HMF** |
| | **PIW** | **Probe** | **(mg/l)** | **PIW** | **Probe** | **(mg/l)** |
| Traubensaft a nativ | 0,276 | 0,787 | **14** | 0,022 | 0,282 | **16** |
| Traubensaft a dotiert 20mg/l HMF | 0,243 | 1,414 | **35** | 0,022 | 0,560 | **34** |
| Traubensaft b nativ | 0,203 | 0,854 | **19** | 0,011 | 0,326 | **20** |
| Traubensaft b dotiert 20mg/l HMF | 0,179 | 1,469 | **39** | 0,011 | 0,618 | **38** |
| Apfelsaft a nativ | 0,138 | 0,159 | **1** | 0,005 | 0,034 | **2** |
| Apfelsaft a dotiert 20mg/l HMF | 0,129 | 0,812 | **21** | 0,005 | 0,334 | **21** |
| Apfelsaft b nativ | 0,134 | 0,142 | **0** | 0,001 | 0,022 | **1** |
| Apfelsaft b dotiert 20mg/l HMF | 0,132 | 0,823 | **21** | 0,001 | 0,330 | **21** |
| Honig 20g in80g Wasser gelöst | 0,146 | 0,345 | **5** | 0,012 | 0,078 | **4** |
| Honig 20g in80g Wasser gelöst dotiert 20 mg/l HMF | 0,129 | 0,897 | **23** | 0,012 | 0,396 | **24** |
| 20 mg HMF in Wasser | 0,133 | 0,819 | **20** | 0,003 | 0,319 | **20** |

### 2. Bestimmung von HMF in Apfelsaft-reflektometrische Auswertung der Reaktionsfarbe:

### Herstellung der Teststäbchen:

Auf ein Filtrierpapier (z.B. Firma Binzer, Typ 1588) wird nachfolgende Tränklösung aufgebracht und danach mit warmer Luft getrocknet.

Das Papier ist mit Schmelzkleber (z.B. Dynapol 1272) auf eine weiße Trägerfolie aufgesiegelt und geeignet in Streifen geschnitten, so dass eine Reaktionszone von ca. 6 mm x 8 mm entsteht.

### Zusammensetzung der Tränklösung:

250 g Pufferlösung (aus 50 g Citronensäure in 1000 g vollentsalztem Wasser gelöst und mit NaOH auf pH 3,5 eingestellt)
250 g Ethanol
25,0 g 4-Amino-phenazon
1,0 g Barbitursäure

### Analyse:

Das Teststäbchen wird für ca. 2 Sekunden in die zu untersuchende Probe oder Standardlösung eingetaucht, kurz abgeschüttelt und im Reflektometer (RQflex^{®}) nach zwei Minuten Reaktionszeit ausgewertet.

Den Zusammenhang zwischen der gemessenen relativen Remission (%) und dem Gehalt an HMF zeigt Tabelle1.

**(Tabelle 3)**

| **HMF in mg/l** | **% Rem** |
|---|---|
| 0 | 81,4 |
| 5 | 71,5 |
| 10 | 63,5 |
| 20 | 52,4 |
| 50 | 36,8 |
| 100 | 25,0 |

### 3. Praxistests

### Praxistest 1:

Mit dem erfindungsgemäßen Verfahren werden verschiedene Lebensmittelproben untersucht und mit dem photometrischen Verfahren nach Winkler verglichen.

Probenvorbereitung:
für helle Säfte: keine.
für Honige: 10 g im Ultraschallbad in 40 g Wasser lösen.

**(Tabelle 4)**

| Probe | photometrische Winklermethode | Erfindungsgemäßes Verfahren mit RQ-Flex^{®} Auswertung |
|---|---|---|
| Standardlösung 5 mg/l HMF | 5,0 | 4,2 |
| Standardlösung 20 mg/l HMF | 21,1 | 18,1 |
| Traubensaft weiß a. | 0,7 | 0,3 |
| Traubensaft weiß b. | 2,1 | 2,1 |
| Apfelsaft a. | 1,4 | 1,6 |
| Apfelsaft b. | 0,4 | 0,3 |
| Apfelsaft c. | 2,4 | 3,0 |
| Waldhonig | 0,3 | 0,3 |
| Akazienhonig | 0,4 | 0,3 |
| Sonnenblumenhonig | 3,6 | 3,1 |

Dotierungsversuche mit 10 mg/l bzw. 10 mg/kg HMF zeigen folgende Ergebnisse:

Die Messungen beider Methoden wurden mit derselben Probe (nativ oder dotiert) durchgeführt.

**(Tabelle 5, Wiederf. bedeutet Wiederfindung)**

| Probe | photometrische Winklermethode | | | Erfindungsgemäßes Verfahren mit RQ-Flex^{®} Auswertung | | |
|---|---|---|---|---|---|---|
| | nativ | dotiert | Wiederf. | nativ | dotiert | Wiederf. |
| Wasser | 0 | 12,0 | 120 % | 0 | 10,3 | 103 % |
| Weißwein a. | 0,3 | 11,0 | 107 % | 0,3 | 8,6 | 81 % |
| Weißwein b. | 2,4 | 14,0 | 116 % | 3,0 | 13,8 | 105 % |
| Weißwein c. | 6,5 | 17,7 | 112 % | 5,9 | 15,8 | 96 % |
| Traubensaft | 13,7 | 25,9 | 122 % | 23,6 | 36,0 | 120 % |
| Apfelsaft | 1,7 | 13,5 | 118 % | 2,1 | 13,4 | 110 % |
| Sonnenblumenhonig | 3,0 | 14,5 | 115 % | 5,0 | 18,3 | 129 % |

## Patentansprüche

1. Mittel zum Nachweis von Furfuralen, **dadurch gekennzeichnet, dass** das Mittel aus einem Teststreifen besteht, der mit einer sauren Lösung imprägniert wurde, die zumindest 0,1 bis 20 Gew.% eines 4-Amino-phenazon-Derivats und 0,05 bis 5 Gew.% eines Barbitursäure-Derivats in einem sauren wässrigen Lösungsmittel mit einem pH-Wert zwischen 2 und 5 enthält.

2. Mittel nach Anspruch 1, **dadurch gekennzeichnet, dass** das Mittel 4-Aminophenazon als 4-Amino-phenazon-Derivat enthält.

3. Mittel nach Anspruch 1 oder 2, **dadurch gekennzeichnet, dass** das Mittel Barbitursäure und/oder Thiobarbitursäure als Barbitursäure-Derivat enthält.

4. Mittel nach einem oder mehreren der Ansprüche 1 bis 3, **dadurch gekennzeichnet, dass** das Mittel Citronensäure enthält.

5. Mittel nach einem oder mehreren der Ansprüche 1 bis 4, **dadurch gekennzeichnet, dass** das Mittel aus einem Teststreifen besteht, der mit einer sauren Lösung imprägniert wurde, die zumindest 0,1 bis 20 Gew.% 4-Aminophenazon und 0,05 bis 5 Gew.% Barbitursäure und/oder Thiobarbitursäure enthält.

6. Mittel nach einem oder mehreren der Ansprüche 1 bis 5, **dadurch gekennzeichnet, dass** das Mittel aus einem Teststreifen besteht, der zusätzlich eine Zone aufweist, die mit einer sauren Lösung getränkt wurde, die zumindest 4-Aminobenzoesäure und ein Barbitursäure-Derivat enthält.

7. Verfahren zum Nachweis von Furfuralen, **gekennzeichnet durch** folgende Verfahrensschritte
a) Bereitstellen einer wässrigen Probenlösung
b) Inkontaktbringen eines Mittels nach einem oder mehreren der Ansprüche 1 bis 6 mit der Probenlösung aus Schritt a)
c) Visuelle und/oder photometrische Auswertung der entstehenden Färbung.

8. Verfahren nach Anspruch 7, **dadurch gekennzeichnet, dass** die Auswertung in Schritt c) reflektometrisch erfolgt.

9. Verwendung des Mittels nach einem oder mehreren der Ansprüche 1 bis 6 zum Nachweis von 5-(Hydroxymethyl)-furan-2-carbaldehyd in Lebensmitteln.

10. Kit zum Nachweis von Furfuralen zumindest enthaltend ein Mittel entsprechend einem oder mehreren der Ansprüche 1 bis 6.

11. Kit nach Anspruch 10, **dadurch gekennzeichnet, dass** der Kit zusätzlich einen Teststreifen enthält, der mit einer sauren Lösung getränkt wurde, die zumindest 4-Aminobenzoesäure und ein Barbitursäure-Derivat enthält.

## Claims

1. Means for the detection of furfurals, **characterised in that** the means consists of a test strip which has been impregnated with an acidic solution which comprises at least 0.1 to 20% by weight of a 4-aminophenazone derivative and 0.05 to 5% by weight of a barbituric acid derivative in an acidic aqueous solvent having a pH between 2 and 5.

2. Means according to Claim 1, **characterised in that** the means comprises 4-aminophenazone as 4-aminophenazone derivative.

3. Means according to Claim 1 or 2, **characterised in that** the means comprises barbituric acid and/or thiobarbituric acid as barbituric acid derivative.

4. Means according to one or more of Claims 1 to 3, **characterised in that** the means comprises citric acid.

5. Means according to one or more of Claims 1 to 4, **characterised in that** the means consists of a test strip which has been impregnated with an acidic solution which comprises at least 0.1 to 20% by weight of 4-aminophenazone and 0.05 to 5% by weight of barbituric acid and/or thiobarbituric acid.

6. Means according to one or more of Claims 1 to 5, **characterised in that** the means consists of a test strip which additionally has a zone which has been impregnated with an acidic solution which comprises at least 4-aminobenzoic acid and a barbituric acid derivative.

7. Method for the detection of furfurals, **characterised by** the following method steps:
a) provision of an aqueous sample solution
b) bringing a means according to one or more of Claims 1 to 6 into contact with the sample solution from step a)
c) visual and/or photometric evaluation of the colouration formed.

8. Method according to Claim 7, **characterised in that** the evaluation in step c) is carried out reflectometrically.

9. Use of the means according to one or more of Claims 1 to 6 for the detection of 5-(hydroxymethyl)furan-2-carbaldehyde in foods.

10. Kit for the detection of furfurals, at least comprising a means corresponding to one or more of Claims 1 to 6.

11. Kit according to Claim 10, **characterised in that** the kit additionally comprises a test strip which has been impregnated with an acidic solution which comprises at least 4-aminobenzoic acid and a barbituric acid derivative.

## Revendications

1. Moyen de détection de furfurals, **caractérisé en ce que** le moyen est constitué d'une bandelette réactive ayant été imprégnée d'une solution acide qui comprend au moins de 0,1 à 20% en poids d'un dérivé de 4-aminophénazone et 0,05 à 5% en poids d'un dérivé d'acide barbiturique dans un solvant aqueux acide ayant un pH compris entre 2 et 5.

2. Moyen selon la revendication 1, **caractérisé en ce que** le moyen comprend de la 4-aminophénazone comme dérivé de 4-aminophénazone.

3. Moyen selon la revendication 1 ou 2, **caractérisé en ce que** le moyen comprend de l'acide barbiturique et/ou de l'acide thiobarbiturique comme dérivé d'acide barbiturique.

4. Moyen selon l'une ou plusieurs parmi les revendications 1 à 3, **caractérisé en ce que** le moyen comprend de l'acide citrique.

5. Moyen selon l'une ou plusieurs parmi les revendications 1 à 4, **caractérisé en ce que** le moyen est constitué d'une bandelette réactive ayant été imprégnée d'une solution acide qui comprend au moins de 0,1 à 20% en poids de 4-aminophénazone et de 0,05 à 5% en poids d'acide barbiturique et/ou d'acide thiobarbiturique.

6. Moyen selon l'une ou plusieurs parmi les revendications 1 à 5, **caractérisé en ce que** le moyen est constitué d'une bandelette réactive possédant en outre une zone ayant été imprégnée d'une solution acide qui comprend au moins de l'acide 4-aminobenzoïque et un dérivé d'acide barbiturique.

7. Méthode de détection de furfurals, **caractérisée par** les étapes de méthodes suivantes :
a) la fourniture d'une solution aqueuse d'échantillon
b) la mise en contact d'un moyen selon l'une ou plusieurs parmi les revendications 1 à 6 avec la solution d'échantillon issue de l'étape a)
c) l'évaluation visuelle et/ou photométrique de la coloration formée.

8. Méthode selon la revendication 7, **caractérisée en ce que** l'évaluation dans l'étape c) est effectuée par voie réflectométrique.

9. Utilisation du moyen selon l'une ou plusieurs parmi les revendications 1 à 6, pour la détection de 5-(hydroxyméthyl)furan-2-carbaldéhyde dans les produits alimentaires.

10. Kit de détection de furfurals, comprenant au moins un moyen correspondant à l'une ou plusieurs parmi les revendications 1 à 6.

11. Kit selon la revendication 10, **caractérisé en ce que** le kit comprend en outre une bandelette réactive ayant été imprégnée d'une solution acide qui comprend au moins de l'acide 4-aminobenzoïque et un dérivé d'acide barbiturique.
